(19) Europäisches Patentamt
European Patent Office
Office européen des brevets

(11) **EP 2 241 921 B1**

(12) **EUROPÄISCHE PATENTSCHRIFT**

(45) Veröffentlichungstag und Bekanntmachung des Hinweises auf die Patenterteilung:
**21.11.2018 Patentblatt 2018/47**

(51) Int Cl.:
**G02B 23/24** *(2006.01)*        **G02B 23/26** *(2006.01)*
**A61B 1/00** *(2006.01)*        **G02B 6/42** *(2006.01)*

(21) Anmeldenummer: **10003569.0**

(22) Anmeldetag: **31.03.2010**

(54) **Endoskopisches oder mikroskopisches System**

Endoscopic or microscopic system

Système endoscopique ou microscopique

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **17.04.2009   DE 102009017940**

(43) Veröffentlichungstag der Anmeldung:
**20.10.2010   Patentblatt 2010/42**

(73) Patentinhaber: **Storz Endoskop Produktions GmbH**
**78532 Tuttlingen (DE)**

(72) Erfinder: **Krattiger, Beat**
**CH-8222 Beringen (CH)**

(56) Entgegenhaltungen:
**WO-A1-2009/021079        WO-A1-2009/034694**
**JP-A- 2005 205 195        US-A- 4 011 403**
**US-A1- 2003 007 714**

- **TOSHIAKI IWAI ET AL: "Speckle Reduction in Coherent Information Processing" PROCEEDINGS OF THE IEEE, IEEE. NEW YORK, US, Bd. 84, Nr. 5, 1. Mai 1996 (1996-05-01), XP011043704 ISSN: 0018-9219**

## Beschreibung

Die Erfindung betrifft ein endoskopisches oder mikroskopisches System zur Beleuchtung eines Operationsgebietes.

Ein derartiges endoskopisches System ist aus der Patentanmeldung JP-2005-205195 bekannt.

Endoskope und Mikroskope stellen in der Medizin inzwischen ein unverzichtbares Hilfsmittel bei bestimmten diagnostischen und therapeutischen Fragestellungen dar. Insbesondere ermöglichen sie im Rahmen der minimal-invasiven Chirurgie eine schonende Alternative zur herkömmlichen offenen Chirurgie. Zur Betrachtung des Untersuchungsgebiets, das normalerweise vollkommen verdunkelt ist, mit Hilfe des Beobachtungssystems eines Endoskops, das als klassisches Relaislinsensystem, als Faserbildleiter oder auch als elektronischer Bildsensor-Chip (CMOS, CCD) zur Video-Bildaufnahme ausgelegt sein kann, ist eine möglichst helle Beleuchtung des Untersuchungsgebiets zwingend erforderlich. Neben der Verwendung in der Human- und Veterinärmedizin haben sich endoskopische oder mikroskopische Untersuchungen auch in technischen Bereichen, beispielsweise zur Untersuchung von Hohlräumen in Turbinen und Motoren, bewährt. Auch bei diesen Anwendungen kann eine gute Ausleuchtung des Untersuchungsgebiets unentbehrlich für eine gute Darstellung durch das Beobachtungssystem des Endoskops sein, die gegebenenfalls auch eine quantitative Auswertung von Risslängen erlauben sollte.

Üblicherweise erfolgt die Beleuchtung des Untersuchungsgebiets über einen Lichtleiter mit geringem Querschnitt, bestehend aus einer optischen Faser oder einem Faserbündel, über welche Licht von einer externen Lichtquelle in das Untersuchungsgebiet geleitet wird.

Die Lichtquelle muss daher Licht mit einer hohen Leistung bzw. Leistungsdichte bereitstellen, um eine ausreichende Beleuchtung der Hohlräume zu gewährleisten.

Dazu werden im Allgemeinen Lichtbogenlampen wie z.B. Hochdruck-Xenon-Kurzbogenlampen verwendet. Diese Lichtbogenlampen strahlen inkohärentes Licht ab, das gegebenenfalls über ein geeignetes Blenden- und Linsensystem auf das Lichteintrittsende der optischen Faser bzw. des Faserbündels abgebildet bzw. fokussiert wird.

Zunehmend kommen aber auch Leuchtdioden und Laser, insbesondere Laserdioden (LD) zum Einsatz. Laserdioden weisen höhere Leuchtdichten als Lichtbogenlampen auf. Laserlichtquellen haben zudem eine hohe Lebensdauer. Durch dünne Sonden und Kabel kann Laserlicht einfach übertragen werden.

Mögliche Laserlichtquellen für Endoskope und OP-Mikroskope können lasergepumpte Fluoreszenzlichtquellen, kontinuierlich modulierte oder gepulste (IR-)Laser , Blau- oder UV-Laser zur Fluoreszenzanregung, kontinuierlich modulierte oder gepulste Blau- oder UV-Laser, RGB- /RGBA-Weisslicht-Laserlichtquellen, Superkontinuum-Weisslichtlaserquellen sowie IR-Laser.

Das Licht, das ein Laser üblicherweise emittiert, weist enge Spektralbanden auf. Durch diese spektrale Reinheit kann Laserlicht insbesondere für Fluoreszenzanregungen bevorzugt verwendet werden, da sich das Anregungslicht somit einfach herausfiltern lässt. Zur Farbwiedergabe ist aber ein weißes Beleuchtungslicht mit einer spektralen Charakteristik, die möglichst einer Schwarzkörperstrahlung angenähert ist, optimal.

In der Patentanmeldung JP 2005-205195 ist daher ein Fluoreszenzumsetzer im Beleuchtungsstrahlengang angeordnet, um nach dem Prinzip der additiven Farbmischung Weißlicht aus dem blauen Anregungslicht eines Lasers und dem in einem Fluoreszenzumsetzer entstehenden gelben Lichtanteilen zu erzeugen. Das aus einer LED oder einer Laserdiode (LD) im blauen Spektralbereich abgestrahlte Licht wird durch eine Kondensoranordnung in eine dünne Multimode-Glasfaser eingespeist. Das andere Ende der Glasfaser ist mit einem Wellenlängen-Umwandlungselement versehen. Dieses besteht aus einem Ausgangsteil am Ende der Glasfaser, das mit Fluoreszenzmaterial umhüllt ist. Wegen der am distalen Ende der Glasfaser konzentrierten Weißlichterzeugung ist die Ausführungsform besonders für endoskopische Anwendungen geeignet. Durch Auswahl der Laser-Emissionswellenlängen und der Komposition des Fluoreszenzmaterials ist eine Vielzahl von Farbabstufungen bei der Fluoreszenzumwandlung und Farbmischung möglich.

Das abgestrahlte Licht von Lasern ist aufgrund seines Entstehungsprozesses kohärent, wohingegen das Fluoreszenzlicht, das der Fluoreszenzkörper aufgrund der Anregung mit Laserlicht aussendet, inkohärent ist.

Der Fluoreszenzumsetzer wird auch als Fluoreszenzkörper bezeichnet, was seine Eigenschaft als Streukörper, der das durchgelassene Anregungslicht in alle Raumrichtungen streut, hervorheben soll. Die Streuung wird durch die im Volumen des Fluoreszenzkörpers eingelagerten Streuzentren und durch Struktureffekte an der Oberfläche bewirkt. Dabei können die Streuzentren gleichzeitig auch die Fluorophore sein. Die Streuzentren können aufgrund ihrer Dimensionierung selektiv die kurzen Wellenlängen bevorzugt streuen.

Durch die Beleuchtung einer Oberfläche mit zumindest teilweise kohärenten Licht können sogenannte Speckle-Muster am Beleuchtunsort entstehen, die in den Aufnahmen des Beleuchtungsorts mit einer Videokamera zu sehen sind.

Die Speckles zeichnen sich häufig durch eine granulare, körnige bzw. sandpapierartige Textur aus. Das Erscheinungsbild eines Speckles kann einer Oberfläche gleichen, die mit feinen Partikeln besprenkelt ist.

Das Auftreten von Speckles ist weniger auffallend und störend bei bewegten, handgehaltenen endoskopischen Instrumenten als bei stationären Instrumenten oder Systemen, wie zum Beispiel einem Operationsmikroskop. Durch die stationäre, unbewegte Position des Operationsmikroskops oder des Zielobjekts werden die Speckles nicht durch die Hand-Zitter-Bewegung (Tremor) zeitlich ausgemittelt und werden somit wesentlich besser erkennbar. Daher ist auch bei OP - Mikroskopen neben den bereits genannten Endoskopen eine Reduktion der Speckles sehr wichtig.

Aus dem Patent US 4,011,403A ist ein Laserbeleuchtungs- und Beobachtungssystem bekannt, bei dem Mittel zur periodische Anregung des Systems mit dem Ziel der Specklesreduktion vorhanden sind.

Eine Reduzierung der Speckle-Muster ist für eine zuverlässige Auswertung der Bildinformation unumgänglich. So könnte beispielsweise bei industriellen Anwendungen, wie der Inspektion von Motorhohlräumen, die scheinbar körnige Textur der Speckle-Artefakte für Korrosion, Verunreinigungen oder Ablagerungen gehalten werden.

Bei medizinischen Anwendungen könnte beispielsweise die Verwechslung der SpeckleMuster mit dem Erscheinungsbild von Läsionen zu Fehldiagnosen bei medizinischen Fragestellungen führen.

In der EP 2 187 259 A1 (WO2009034694) sind Vorrichtungen beschrieben, mit denen unter anderem Specklemuster oder Lichtgranulation durch eine zeitliche Mittelung von Specklemustern verhindert werden. Dazu wird eine Projektionslinse mittels eines piezoelektrischen Elements oder eines Ultraschallmotors vibriert. Ferner sind das Vibrieren eines holografischen Diffusors mittels eines piezoelektrischen Elements oder eines Ultraschallmotors, das Vibrieren der Ausgangsfläche eines Faserbündels und das Vibrieren der Eingangsfläche eines Faserbündels beschrieben.

In dem Artikel "Speckle Reduction in Coherent Information Processing" von Toshiaki Iwai und Toshimitsu Asakura (Proceedings of the IEEE, IEEE, New York, Band 84, Nr. 5, Mai 1996) ist die Reduktion von Speckle-Mustern durch zufällige Vibration einer Lichtleitfaser beschrieben.

Davon ausgehend liegt der Erfindung die Aufgabe zugrunde, ein endoskopisches oder mikroskopisches System der eingangs genannten Art dahingehend weiterzubilden, dass es eine Beleuchtung möglichst ohne Speckles am Untersuchungsort effizient ermöglicht.

Hinsichtlich des eingangs genannten endoskopischen oder mikroskopischen Systems wird die Aufgabe dadurch gelöst, dass eine aperiodische Störung des zumindest einen Lichtleitelements und/oder der mindestens einen Lichtquelle mittels eines Aktors dazu verwendet wird, eine möglichst wirksame und effiziente Reduktion von Speckles zu erreichen.

Der Begriff "Störung" im Sinne der Erfindung umfasst die aperiodische, chaotische und stochastische Veränderung einer physikalischen Größe sowie auch Veränderungen einer physikalischen Größe, die sonstige zufällige Komponenten in ihrem Zeitverlauf umfassen. Insbesondere sind mechanische Bewegungen, Auslenkungen, Vibrationen, Oszillationen oder Zitterbewegungen unter dem Begriff Störung zu verstehen. Die mechanischen Bewegungen können aperiodische, chaotische, regellose, unvorhersehbare oder mit sonstigen zufälligen Komponenten behaftete Zeitverläufe aufweisen. Der Zeitverlauf einer mechanischen Bewegung kann sich aber auch aus einem periodischen, wiederkehrenden Anteil und einem aperiodischen, chaotischen, regellosen, unvorhersehbaren oder mit sonstigen zufälligen Komponenten behafteten Anteil zusammensetzen und dadurch insgesamt aperiodisch sein. Hierdurch wird erfindungsgemäß ein breites Frequenz- und Amplitudenanregungsspektrum der Störung erzielt, was zu einer äußerst effizienten Reduktion von Speckles führt.

Die Störungen können eine Änderung des Brechungsindex des Ausbreitungsmediums bewirken, in dem das Licht transportiert wird. Bei mechanischen Vibrationen entstehen mechanische Spannungsunterschiede die Änderungen des Brechungsindex hervorrufen. Dadurch wird die Phase des Lichts moduliert, wodurch dessen Kohärenz reduziert werden kann. Bei der Verwendung einer Pockels- oder Kerr-Zelle wird durch Anlegen einer Spannung an die Elektroden der Zelle ein elektrisches Feld erzeugt, das eine Änderung des Brechungsindex hervorrufen kann. Durch diese Änderungen des Brechungsindex des Ausbreitungsmediums kann wiederum die Phase des kohärenten Lichts moduliert und dessen Kohärenz reduziert werden. Die reduzierte Kohärenz ermöglicht eine zeitliche Ausmittlung der Speckles, wodurch deren Auftreten und Intensität reduziert wird.

Der Begriff "Aktor" bezeichnet im Sinne der Erfindung einen Wandler, der elektrische Signale beispielsweise in mechanische Bewegung umsetzt. Aktoren können direkt physikalische Größen beeinflussen wie Temperatur, Druck und Dichte von Gasen, Lage und Bewegung von Körpern im Raum. Sie können aber auch elektromagnetische Erscheinungen hervorrufen wie beispielsweise elektromagnetische Wellen und Impulse.

Als Aktoren sind beispielsweise Elektromotoren, elektrodynamische Wandler und elektrostrikitve, oder magnetostriktive Aktoren, insbesondere Niedervolt-Piezo-Aktoren, zu nennen. Als bevorzugte Elektromotoren sind Vibrationsmotoren, die in Mobiltelefonen, zur Auslösung des taktilen Alarmes eingesetzt werden, zu erwähnen. Aufgrund ihrer platzsparenden Abmessungen eignen sie sich bestens für den Einsatz in einem endoskopischen System. Darüber hinaus sind sie wegen ihrer massenhaften Herstellung sehr kostengünstig. Aktoren, welche elektrische Felder als physikalische Größen beeinflussen, stellen beispielsweise die Elektroden einer Pockels- oder Kerr-Zelle dar.

Die Begriffe "Lichtleitelement" oder "Lichtleiter" im Sinne der Erfindung umfassen alle dem Fachmann bekannten Mittel, die zur Übertragung des Lichts der Lichtquelle zum distalen Ende eines Endoskops oder Mikroskops angewandt werden. Diese umschließen beispielsweise optische Fasern, Singlemode-Fasern, Multimode-Fasern, Faserbündel, Lichtleiterkabel, Linsen, Stablinsen, Blenden, Filter, Lichtkonversionselemente, Lichtstecker, Spiegel, Faserkegel, Glaskegel, Glaskörper, Kristallkörper, Lichtschächte sowie auch Lichtkuppler zum Anschluss der Lichtleitfaser(n) an die Lichtquelle.

Der Begriff "Kopplung" im Sinne der Erfindung bezeichnet jede Verbindung, die eine zumindest teilweise Übertragung der durch den Aktor erzeugten Energie gewährleistet. Die Kopplung kann als starre Verbindung insbesondere als Stoff-, form- oder kraftschlüssige Verbindung mit Hilfe eines Kopplungselements realisiert sein, was eine permanente Übertragung mechanischer Bewegungsenergie erlaubt. Die Kopplung kann aber auch in Form einer losen Verbindung, die nur zeitweise besteht und keine vollständige Übertragung der mechanischen Energie des Aktors bewirkt, ausgebildet sein. Sie kann auch in Form einer Spule ausgebildet sein, die elektromagnetische Impulse und somit elektromagnetische Energie aufnimmt.

Die im Sinne der Erfindung verwandten Lichtquellen zur Erzeugung von zumindest teilweise kohärenten Licht können auch spektrale Anteile des ultravioletten oder des infraroten Spektralbereichs des elektromagnetischen Spektrums umfassen.

Beschreibung bevorzugter Ausführungen

In einer ersten Ausgestaltung der Erfindung sind die Lichtquelle und der Aktor gemeinsam auf einem Kühlkörper montiert, der neben dem Abtransport der Abwärme der Lichtquelle die Übertragung der mechanischen Anregungen des Aktors auf die Lichtquelle bewirkt. Der hierzu elastisch aufgehängte Kühlkörper dient somit zusätzlich zu seiner ursprünglichen Funktion als Kopplungselement. Der Aktor ist mit einer Steuerschaltung versehen, die den Aktor mit einem chaotischen Eingangssignal speist. Der Aktor wandelt das elektrische Eingangssignal in entsprechende mechanische Bewegungen, die den Zeitverlauf des chaotischen Eingangssignals besitzen. Die mechanischen Bewegungen werden über den Kühlkörper auf die Lichtquelle übertragen und regen diese zu chaotischen Bewegungen mit geringen Auslenkungen um ihre Ruhelage an.

Durch diese Maßnahme entfällt vorteilhafterweise die Notwendigkeit für ein zusätzliches Bauteil zur Kopplung des Aktors an die Lichtquelle, was Materialkosten spart.

Ein weiterer Vorteil der Anordnung des Aktors auf dem Kühlkörper ist, dass ein Austausch des Aktors und der zugehörigen Steuerungselektronik besonders einfach und zeitsparend durchgeführt werden kann. Da der Aktor und die Steuerungselektronik frei zugänglich auf dem Kühlkörper angeordnet sind, wird während der Reparatur keine zusätzliche Zeit zum Aus- bzw. Einbauen des Kühlkörpers benötigt.

In einer weiteren bevorzugten Ausgestaltung ist der Aktor mit einem Lichtleitelement direkt verbunden. Hierzu kann der Aktor beispielsweise einen Linearantrieb oder einen Kurbeltrieb mit Antriebsstange aufweisen, die eine lochförmige Öffnung zum Beispiel als Bohrung senkrecht zur Antriebsrichtung aufweist, durch jene eine Lichtleitfaser bzw. ein Faserbündel als Lichtleitelement formschlüssig oder lose eingefädelt wird.

Durch diese Maßnahme kann in vorteilhafterweise eine Dejustierung der Lichtquelle, ihrer Abbildungsoptik sowie weiterer abbildenden optischen Bauteile innerhalb des endoskopischen oder mikroskopischen Systems durch die mechanischen Störungen, die durch den Aktor im endoskopischen oder mikroskopischen System erzeugt werden, vermieden werden.

In einer bevorzugten Ausgestaltung verbindet ein zu Drehschwingungen anregbares Kopplungselement den Aktor mit dem Lichtleiter.

Diese Maßnahme kann in vorteilhafterweise leicht realisiert werden, da ein zu Drehschwingungen anregbares Kopplungselement in Form einer Unruh eine massenhafte Herstellung in der Uhrenindustrie erfährt und daher leicht und kostengünstig beziehbar ist. Darüber hinaus sind Unruhen und ihre Aktoren durch die stetigen Entwicklungen der Uhrenindustrie in miniaturisierter Bauweise erhältlich, die den Erfordernissen zur Raumeinsparung bei der Verwendung in einem Endoskop oder Mikroskop angepasst sind.

In einer weiteren bevorzugten Ausgestaltung weist das Kopplungselement ein Dämpfungselement auf.

Diese Maßnahme hat den Vorteil, dass die durch das Kopplungselement übertragene Anregungsenergie in seiner Wirkung begrenzt werden kann, um einer unbeabsichtigten Verstärkung der Störungen im endoskopischen oder mikroskopischen System beispielsweise in Form einer Resonanzkatastrophe vorzubeugen.

In einer weiteren bevorzugten Ausgestaltung weist der Aktor eine Unwucht auf.

Durch diese Maßnahme kann vorteilhafterweise auf eine gesonderte Steuerungsschaltung, die beispielsweise ein chaotisches Eingangssignal für den Aktor erzeugt, verzichtet werden. Stattdessen kann der Aktor mit einer gewöhnlichen Gleich- oder Wechselspannung betrieben werden. Der Aktor erzeugt eine periodische modulierte Anregungsenergie, die über das Kopplungselement, aufgrund seiner Eigenschaft das Lichtleitelement in eine chaotische mechanische Bewegung versetzt. Dadurch werden die Kosten und der Aufwand für eine elektronische Steuerungsschaltung, die eine zufällige Komponente erzeugt, eingespart. Die zufällige Komponente, die als Störung auf das endoskopische oder mikroskopische System übertragen wird, entsteht durch die mechanische Ausgestaltung der Kopplung. Es bedarf keiner Steuerungsfunktion, die eine zufällige Komponente umfasst. Durch die zufällige Komponente entsteht zudem eine Störung mit einem breitem Frequenz- und Amplitudenspektrum, die in vorteilhafter Weise eine effizientere Reduktion von Speckles ermöglicht.

In vorteilhafter Weise kann der Aktor als Kühllüfter mit Unwucht ausgeführt sein, wobei die Unwucht des Kühllüfters durch lokales Beschweren oder Entfernen eines Teils eines Flügelblatts des Kühllüfters insbesondere durch Wegbrechen erzeugt wird.

Durch diese Maßnahme steht ein kostengünstiger, leicht verbaubarer und austauschbarer Aktor mit Unwucht als kompakte Baugruppe zur Verfügung.

In einer besonders bevorzugten Ausgestaltung wird durch die Kopplung eine lose Verbindung zwischen Aktor und Lichtleiter hergestellt. Als Kopplungselement kann beispielsweise eine Schlaufe, ein Ring, eine Schlinge, eine Öse, eine Antriebsstange mit Bohrung oder ein schraubenförmig gebogener Draht dienen. Durch die Öffnung des Kopplungselements wird der Lichtleiter durchgeführt. Der Lichtleiter kann sich lateral innerhalb des Raums, der durch die Ränder des Kopplungselements umschlossen wird, frei bewegen. Der Aktor führt beispielsweise mechanische Bewegungen aus, deren Auslenkungen größer oder gleich dem größten Durchmesser des Querschnitts der Öffnung des Kopplungselements sind. Der Lichtleiter erfährt aber auch bei Auslenkung des Kopplungselements durch den Aktor einen Stoß bzw. einen Schlag, falls der Abstand des Lichtleiters zu den Rändern der Öffnung kleiner als die Auslenkung des Kopplungselements ist. Verhältnismäßig kleine Auslenkungen bewirken beispielsweise dann eine Schlaganregung, wenn der Lichtleiter das Kopplungselement berührt oder mit einer verschwindend kleinen Kraft gegen den Lichtleiter gedrückt wird. Da sich die relative Position zwischen Lichtleiter und den Rändern der Öffnung des Kopplungselements andauernd in einer unvorhersehbaren Weise ändert, erfährt der Lichtleiter eine Abfolge von Stößen, die rein zufälliger Natur ist. Zur Erzeugung dieser zufälligen Schlaganregung ist keine Steuerung vonnöten, die eine zufällige Komponente beinhaltet. Vorteilhaft ist bei dieser Ausgestaltung der Kopplung auch, dass sie besonders kostengünstig und einfach realisiert werden kann und zudem eine aperiodische Störung ermöglicht.

In einer weiteren bevorzugten Ausgestaltung sind die Lichtquelle, das optische Lichtleitelement und der Aktor in der proximalen Versorgungseinheit angeordnet.

Diese Maßnahme hat den Vorteil, dass das endoskopische oder mikroskopische System eine kompakte Bauweise aufweist, die technisch einfach zu realisieren ist. Ferner erweist sich diese Ausgestaltung des endoskopischen oder mikroskopischen Systems während des Betriebs als vorteilhaft, da das endoskopische oder mikroskopischen System nur das Gehäuseteil der proximalen Versorgungseinheit umfasst, und nicht aus mehreren Gehäuseteilen besteht, so dass beispielsweise bei Inbetriebnahme des endoskopischen oder mikroskopischen Systems keine Schwierigkeiten auftreten können, die aus einem Zusammenbau von mehreren Gehäuseteilen resultieren.

In einer weiteren bevorzugten Ausgestaltung ist das optische Lichtleitelement elektrisch und/oder thermisch isolierend ausgebildet.

Diese Maßnahme hat den Vorteil, dass kein Stromfluss von der mit Spannung beaufschlagten Lichtquelle auf das optische Lichtleitelement, d.h. auf das Lichtleiterkabel und das Gehäuse, überführbar ist. Dies erhöht die Sicherheit der Person, die das endoskopische oder mikroskopische System während beispielsweise einer medizinischen Untersuchung verwendet. Ferner kann sich das Lichtleitelement nicht durch den eingespeisten Strom erwärmen, so dass es nicht durch Aufheizung in seiner Funktionsweise beeinträchtigt wird.

In einer weiteren bevorzugten Ausgestaltung ist das optische Lichtleitelement als Glaskörper, insbesondere als Linse, ausgebildet.

Diese Maßnahme ermöglicht vorteilhafterweise eine ausreichend aus dem Stand der Technik bekannte Ausgestaltungsform des optischen Lichtleitelements, um das von der Lichtquelle emittierte Licht zu sammeln und gegebenenfalls in weitere lichtleitende Elemente, wie Faserkegel, Glaskegel, Lichtleitfasern und Lichtleiterkabel, einzukoppeln. Ferner ist ein Glaskörper besonders kostengünstig herzustellen und gewährleistet aufgrund seiner geringen elektrischen Leitfähigkeit eine elektrische Isolierung zwischen der Lichtquelle und dem Lichtleiterkabel. Das Lichtleitelement kann beispielsweise auch als Compound Elliptical Concentrator (CEC) oder als Compound Hyperbolic Concentrator (CHC) ausgebildet sein.

In einer weiteren alternativen Ausgestaltung ist das optische Lichtleitelement aus optischen Lichtleitfasern aufgebaut.

Diese Maßnahme bietet ebenso eine vorteilhafte Ausgestaltung des optischen Lichtleitelements. Die Verwendung von Lichtleitfasern vermeidet eine aufwändige Oberflächenform des Glaskörpers zur Lichtleitung, da die von der Lichtquelle emittierten Lichtstrahlen durch die Ausrichtung der optischen Lichtleitfaser durch das optische Lichtleitelement geleitet werden. Ferner ermöglicht die Verwendung von Lichtleitfasern ein Ausnutzen der gesamten zur Stirnseite eines gegebenenfalls nachfolgend angeordneten Lichtleiterkabels hingewandten Oberfläche des distalen Endbereichs des Lichtleitelements, um die Lichtstrahlen in das Lichtleitkabel einzukuppeln.

In einer weiteren bevorzugten Ausgestaltung verjüngt sich das optische Lichtleitelement in Richtung zur Lichtquelle hin.

Diese Maßnahme hat den Vorteil, dass die Querschnittsfläche des ersten Endbereichs des optischen Lichtleitelements optimal an die Querschnittsfläche der Lichtquelle oder des Fluoreszenzumsetzers angepasst werden kann und folglich eine effiziente Einkopplung des Lichts in das optische Lichtleitelement ermöglicht wird. Ferner werden die Lichtstrahlen im optischen Lichtleitelement aufgrund seiner sich zu einem gegebenenfalls nachgeordneten Lichtleitelement hin aufweitenden Ausgestaltung aufgeweitet, so dass die Stirnseite des gegebenenfalls nachgeordneten Lichtleitelements, das gewöhnlich einen größeren Durchmesser als die aktive Fläche der Lichtquelle oder des Fluoreszenzumsetzers aufweist, optimal beleuchtet wird.

In einer weiteren bevorzugten Ausgestaltung ist die mindestens eine kohärente Lichtquelle als Halbleiterlaser, insbesondere Diodenlaser ausgebildet.

Diese Maßnahme hat den Vorteil, dass mit der Entwicklung von immer kostengünstigeren und leistungsstärkeren Diodenlasern diese als effiziente Lichtquellen im Bereich der Endoskopie oder Mikroskopie eingesetzt werden können.

In einer weiteren bevorzugten Ausgestaltung umfasst die Lichtquelle mehrere Diodenlaser, die Licht unterschiedlicher Wellenlänge in verschiedenen Bereichen des elektromagnetischen Spektrums, insbesondere im sichtbaren Bereich, emittieren.

Diese Maßnahme hat den Vorteil, dass durch das Einkoppeln von Laserlicht verschiedener Wellenlänge eine spektrale Anpassung an die Erfordernisse zur Beobachtung der Szenerie am Untersuchungsort erzielt werden kann.

In einer weiteren bevorzugten Ausgestaltung ist ein Fluoreszenzumsetzer am distalen Ende des Einführteils angeordnet und wird mittels eines Aktors zur Reduktion von Speckles erfindungsgemäß zu Schwingungen angeregt.

Diese Maßnahme hat den Vorteil, dass die Wirkung des Fluoreszenzumsetzers als Streukörper in vorteilhafter Weise ausgenutzt werden kann. Das emittierte Fluoreszenzlicht des Fluoreszenzwandlers wird in alle Raumrichtungen abgegeben. Darüber hinaus ist der Fluoreszenzwandler als Streukörper für das zumindest teilweise kohärente Licht der mindestens einen Lichtquelle geeignet und kann dieses in alle Raumrichtungen streuen. Dadurch kann eine gleichmäßigere Ausleuchtung von Hohlräumen erzielt werden.

In einer weiteren bevorzugten Ausgestaltung ist der Fluoreszenzumsetzer in der proximalen Versorgungseinheit angeordnet.

Diese Maßnahme hat den Vorteil, dass dadurch eine gute Kühlung des Fluoreszenzumsetzers in der proximalen Versorgungseinheit, die als separate Beleuchtungseinheit eingesetzt werden kann, erreichbar wird. Durch diese modulare Bauweise kann die proximale Versorgungseinheit auch mit verschiedenen Einführungsteilen verwendet werden, was die Flexibilität bei der Zusammenstellung und dem Aufbau eines endoskopischen oder mikroskopischen Systems erhöht.

In einer weiteren bevorzugten Ausgestaltung ist der Aktor als ein Vibrationsmotor ausgebildet, der üblicherweise zur Auslösung des taktilen Alarms in Mobilfunktelefonen verwendet wird.

Diese Maßnahme hat den Vorteil, dass solche Vibrationsmotoren kostengünstig verfügbar sind.

In einer weiteren bevorzugten Ausgestaltung ist der Aktor ein elektromechanischer Wandler, wie er zum Antrieb einer Unruh in einer Uhr verwendet wird.

Diese Maßnahme hat den Vorteil, dass die in der Uhrenindustrie verwandten elektromechanischen Wandler eine miniaturisierte Bauweise aufweisen, die sich zum Einbau im proximalen Bedienteil beispielsweise eines Endoskops aufgrund der Notwendigkeit zur Raumersparnis besonders eignet.

In einer weiteren bevorzugten Ausgestaltung ist der Aktor ein Ventilator.

Diese Maßnahme hat den Vorteil, dass neben den mechanischen Anregungen, die vom Ventilator ausgekoppelt werden und zur Erzeugung von Störungen im endoskopischen oder mikroskopischen System verwendet werden können, auch dessen erzeugter Luftstrom zur Kühlung der Lichtquelle verwendet werden kann.

In einer weiteren bevorzugten Ausgestaltung ist der Aktor ein handelsüblicher Lautsprecher mit vorgeschaltetem Frequenzgenerator.

Diese Maßnahme hat den Vorteil, dass Lautsprecher kostengünstig erworben werden können und leicht in ein Gehäuse eingebaut werden können. Durch den vorgeschalteten Frequenzgenerator können Signale mit Frequenzen, die beispielsweise größer als 20 kHz sind erzeugt werden. Diese Frequenzen sind für das menschliche Ohr nicht mehr wahrnehmbar. Der Lautsprecher wird mit den Signalen mit Frequenzen größer 20 kHz gespeist. Dadurch wird die Membran des Lautsprechers mit Frequenzen, die im Wesentlichen größer als 20 kHz sind, zu Schwingungen angeregt.

In einer alternativen Ausgestaltung wird der Lautsprecher bei tieferen Frequenzen im Bereich 10-1000 Hz angeregt. Dadurch kann mit im wesentlichen gleicher Kraft eine größere Schwingungsamplitude erzeugt werden, wodurch eine bessere Ausmittlung und damit Reduktion der Speckles erzielt werden kann.

Eine besonders vorteilhafte Ausgestaltung des Lautsprechers ergibt sich, wenn der Lautsprecher offen ist.

Bei der offenen Ausführung des Lautsprechers werden die an der Vorderseite der Lautsprechermembran erzeugten Druckwellen im wesentlichen vom Gegendruck der Membranrückseite kompensiert. Dadurch wird bei tiefen Frequenzen, d.h. Frequenzen, die kleiner oder gleich dem Quotienten aus Schallgeschwindigkeit und Lautsprecherdurchmesser sind, kaum hörbarer Schall abgestrahlt. Somit wird in günstiger Weise die Abstrahlung von Störschall minimiert, was dazu beiträgt die Lärmbelastung der Umgebung gering zu halten.

In einer weiteren bevorzugten Ausgestaltung weist die proximale Versorgungseinheit des endoskopischen Systems ein elektrisch isolierendes Halteelement auf, in dem das optische Lichtleitelement angeordnet ist.

Diese Maßnahme ermöglicht vorteilhafterweise eine weitere Isolation der mit Spannung beaufschlagbaren Lichtquelle von dem optischen Lichtleitelement und dem Gehäuse der proximalen Versorgungseinheit, so dass ebenfalls eine Personengefahr verringert wird.

In einer weiteren bevorzugten Ausgestaltung weist die proximale Versorgungseinheit des endoskopischen oder mikroskopischen Systems ein elektrisch isolierendes und Schall dämpfendes Halteelement auf, in dem der Aktor angeordnet ist.

Diese Maßnahme ermöglicht vorteilhafterweise eine Minimierung der mechanischen Störungen, die unbeabsichtigt auf andere Teile des endoskopischen oder mikroskopischen Systems als das anzuregende Lichtleitelement übertragen werden können. Ferner kann durch diese Maßnahme eine Schalldämpfung der unerwünschten Nebengeräusche, die beim Betrieb des Aktors entstehen können, erzielt werden.

In einer weiteren bevorzugten Ausgestaltung weist die proximale Versorgungseinheit des endoskopischen oder mikroskopischen Systems ein reibungsarmes Haltelement auf, in dem das Kopplungselement angeordnet wird.

Durch diese Maßnahme wird vorteilhafterweise eine möglichst verlustfreie Übertragung der Energie des Aktors auf das Lichtleitelement bewerkstelligt.

Weitere Vorteile der Merkmale ergeben sich aus der nachfolgenden Beschreibung und der beigefügten Zeichnungen. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden in der nachfolgenden Beschreibung näher erläutert. Es zeigen:

Fig. 1 eine schematische Darstellung eines endoskopischen Systems

Fig. 2 eine schematische Darstellung der proximalen Versorgungseinheit in Fig. 1

Fig. 3 eine detaillierte Darstellung der proximalen Versorgungseinheit in Fig. 1

Fig. 4 eine schematische Darstellung einer Versorgungseinheit mit proximal angeordneten Fluoreszenzumsetzer

Fig. 5 ein Aktor mit bevorzugtem Kopplungselement und Lichtleitelement

Fig. 6 eine weitere Darstellung eines Aktors mit Kopplungselement und Lichtleitelement

Fig. 7 an ein Lichtleitelement stoffschlüssig gekoppelter Aktor

Fig. 8 eine Aufhängung für einen in einer proximalen Versorgungseinheit angeordneten Aktor

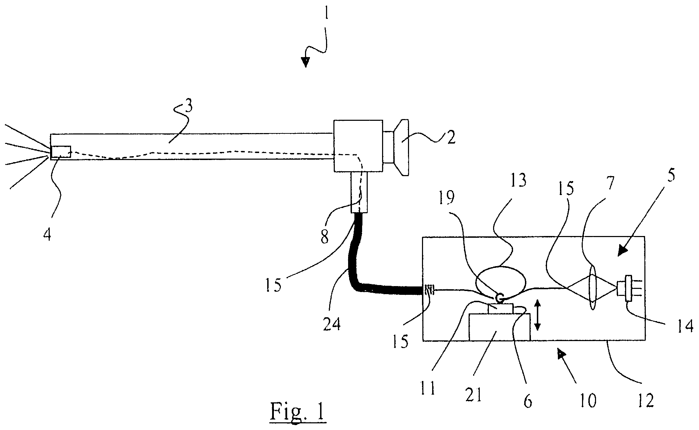

Fig. 1 zeigt schematisch ein endoskopisches System 1 mit Okular 2 und Einführungsteil 3. Das Einführungsteil 3 kann als starres Rohr oder flexibel ausgebildet sein. Nach oder anstelle des Okulars mit optischer Übertragung des beobachteten Bildes kann auch eine Videokamera mit Darstellung des beobachteten Bildes auf einem Monitor vorgesehen sein. In einer Versorgungseinheit 10 ist eine Anregungsstrahlenquelle 5 angeordnet, die eine Laserdiode 14 und eine Kupplungsoptik 7 zur Einspeisung des Anregungslichts in einen Lichtleiter 13, der als Glasfaser ausgebildet ist, enthält. Die Einkopplung des Anregungslichts kann auch direkt ohne Zwischenschaltung einer Kupplungsoptik durch Quasikontaktierung der Oberfläche des Chips der Laserdiode mit dem proximalen Ende der Glasfaser 13 erfolgen, wobei gegebenenfalls ein Minimalabstand zum Austrittsspiegel des LaserChips für eine ausreichende Rückkopplung ins Laser-Medium garantiert werden muss. Selbstverständlich ist es auch möglich, weitere Laserdioden mit Emission zusätzlicher Wellenlängen vorzusehen, deren Strahlung ebenfalls in den Lichtleiter 13 oder in zusätzliche Glasfasern eingespeist werden kann. Damit können z.B. spektrale Schwächen des Weißlichts ausgeglichen werden. Die Laserdioden können batteriebetrieben sein oder über ein Netzteil mit Energie versorgt werden.

Die Glasfaser 13 wird durch ein Kopplungselement 19, das als metallische Öse ausgebildet ist, durchgeführt. Die metallische Öse 19 ist an das Gehäuse 6 des Aktors 11, der als Vibrationsmotor ausgebildet ist, angeschweißt. Der Vibrationsmotor 11 ist auf einem Haltelement 21 angeordnet, das an dem Gehäuse 12 der Versorgungseinheit 10 befestigt ist. Das Haltelement 21 ist zumindest teilweise aus Materialien gefertigt, die eine hohe Elastizität und/oder eine hohe Dämpfungswirkung besitzen. Durch eine entsprechende, geeignete Auswahl der Materialkomponenten des Halteelements 21 kann eine Übertragung der mechanischen Bewegungen, die der Vibrationsmotor 11 erzeugt, auf das Gehäuse 12 der Versorgungseinheit 10 verringert werden. Das Haltelement 21 übt eine vergleichbare Wirkung wie ein aus dem Fahrzeugbau bekannter Stoßdämpfer aus. Durch die Bewegungen des Vibrationsmotors 11 wird die Öse 19 gegen die Glasfaser 13 geschlagen. Die Reservelänge der Glasfaser 13 kann zu einer Spirale geformt sein. Dadurch kann ein größerer Teil der Glasfaser 13 zu mechanischen Bewegungen angeregt werden, ohne dabei die Amplituden der Vibrationsbewegungen des Vibrationsmotors 11 erhöhen zu müssen. In Fig.1 zeigt der Doppelpfeil die Richtung der Bewegungen an, die der Aktor 11 im dargestellten Ausführungsbeispiel ausführt.

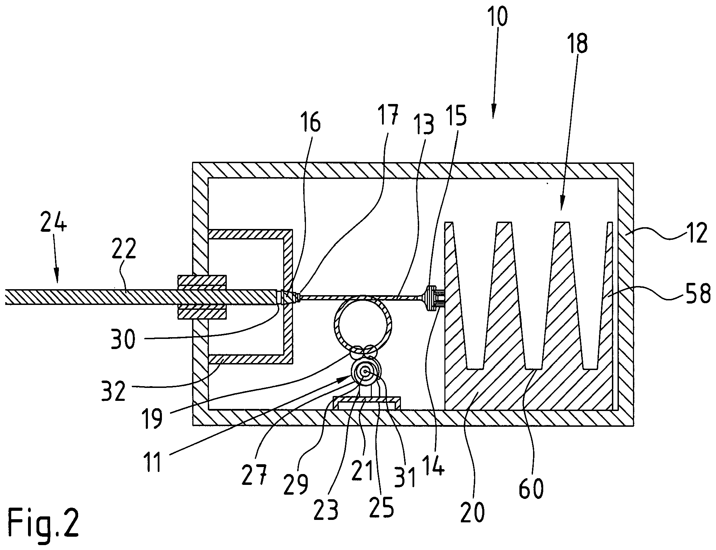

Zur Verbindung der Versorgungseinheit 10 mit dem endoskopischen System 1 ist ein Lichtleiterkabel 24 vorgesehen, das über spezielle oder handelsübliche Steckverbinder am Endoskop oder beispielweise mit einem Lichtleitstecker 15 an der Versorgungseinheit 10 angeschlossen sein kann. Es sind aber auch andere Verbindungsarten denkbar, wie im Detail in Fig. 2 ausgeführt wird. Die Steckverbinder können insbesondere autoklavierbar und lasergeschützt ausgeführt sein. Durch das Einführungsteil 3 hindurch wird die Glasfaser 8 üblicherweise lose oder in einem separaten Beleuchtungskanal oder in einer Schutzhülle - zum distalen Ende geführt. Am distalen Ende ist ein Fluoreszenzumsetzer 4 angeordnet, in dem die Umwandlung in Weißlicht erfolgt. Der Fluoreszenzumsetzer 4 kann funktional austauschbar sein oder in einen austauschbaren Wechselkopf am distalen Ende des Einführungsteils integriert sein. Die Abbildungsoptik ist hier nicht weiter dargestellt.

In Fig. 2 ist die Versorgungseinheit 10 des endoskopischen Systems 1 dargestellt. Die Versorgungseinheit 10 weist ein Gehäuse 12 auf, in dem eine Lichtquelle 14, ein Lichtleiter 13, ein Aktor 11, Kopplungselement 19 und eine Kühlvorrichtung 18 angeordnet sind.

Die Lichtquelle 14 ist thermisch leitend mit der Kühlvorrichtung 18, d.h. mit einem wärmeleitfähigen Kühlkörper 20, verbunden, wobei unter einer thermisch leitenden Verbindung eine direkte oder indirekte thermische Kopplung der beiden Bauteile zu verstehen ist. Der Kühlkörper 20 ist an einer vom optischen Lichtleiter 13 abgewandten Seite der Lichtquelle 14 angeordnet, wobei die Lichtquelle 14 vorzugsweise direkt am Kühlkörper 20 angeordnet ist, so dass von der Lichtquelle 14 erzeugte Wärme direkt an den Kühlkörper 20 abgegeben und von diesem abgeführt werden kann. Der Kühlkörper 20 ist ferner thermisch leitend mit dem Gehäuse 12 verbunden, so dass die vom Kühlkörper aufgenommene Wärme an das Gehäuse 12 abgeführt werden kann. Der Kühlkörper 20 ist bezüglich seiner Abmessungen groß gegenüber der Lichtquelle 14 ausgebildet, so dass der Kühlkörper 20 effizient die von der Lichtquelle 14 erzeugte Wärme aufnehmen und abführen kann.

Der Aktor 11 ruht auf einem elastischen Tragelement 23, das auf einem Halteelement 21 befestigt ist. Das Halteelement ist direkt mit dem Gehäuse 12 verbunden. Im Gehäuse 25 des Aktors 11 ist ein Motor 27 mit einem Unwuchtelement 29 angeordnet. Bei Betrieb des Motors 27 rotiert das Unwuchtelement, das in Form einer Exzenterscheibe ausgebildet ist, um die Motorwelle 31. Durch die Drehbewegung des Unwuchtelements wird das Gehäuse 25 des Aktors 21 ausgelenkt. Diese Auslenkungen übertragen sich direkt auf die Kopplungselemente 19, die stoffschlüssig mit dem Gehäuse 25 verbunden sind. Das elastische Tragelement 23 verhindert eine Übertragung der Auslenkungen auf das Halteelement 21, wodurch sich die Auslenkungen auf das Gehäuse 12 und damit in der gesamten Versorgungseinheit ausbreiten könnten und zu unbeabsichtigten Störungen des endoskopischen Systems 1 führen würden.

Das Lichtleitelement 13 ist als Lichtleitfaser ausgebildet. Diese ist durch einen Lichtleitstecker 15 mit der Lichtquelle 14, die in Form eines Diodenlasers ausgebildet ist, lichtleitend verbunden ist. Das Lichtleitelement 13 wird durch die größeren Öffnungen der Kopplungselemente 19, die in Form von Metallringen ausgebildet sind, durchgeführt. Die Kopplungselemente 19 stoßen bei Betrieb des Motors 27 von Zeit zu Zeit in unvorhersehbarer Weise gegen das Lichtleitelement 13, da die Exzenterscheibe 29, die um die Motorenachse 31 rotiert, das Gehäuse 25 des Aktors 11 und die darauf angeordneten Kopplungselemente auslenkt. Dadurch wird der Verlauf des Strahlengangs in der Lichtleitfaser geändert, wodurch die Kohärenz des Lichts der Laserdiode 14 am distalen Übergang der Lichtleitfaser 13 zum optischen Lichtleitkopplungselement 17 zerstört wird. Das Lichtleitkopplungselement 17 stellt eine lichtleitende Verbindung zwischen der Lichtleitfaser 13 und dem optischen Lichtleitelement 16 her.

Ein proximales Ende 22 eines Lichtleiterkabels 24, das mit einem Einführungsteil (vgl. Fig. 1) verbunden ist, ist durch eine in einer Öffnung 26 des Gehäuses 12 angeordneten Buchse 28 in das Gehäuse 12 einführbar bzw. das proximale Ende 22 des Lichtleiterkabels 24 kann in einem eingeführten Zustand fest in der Versorgungseinheit 10 angeordnet sein. Das proximale Ende 22 des Lichtleiterkabels 24 kommt derart in dem Gehäuse 12 zu liegen, dass eine Stirnfläche 30 des proximalen Endes 22 des Lichtleiterkabels 24 in Richtung der Lichtquelle 14 weist und nahe von dieser beabstandet angeordnet ist. Zwischen der Stirnfläche 30 des proximalen Endes 22 des Lichtleiterkabels 24 und der Lichtquelle 14 ist das elektrisch isolierende optische Lichtleitelement 16 angeordnet, das durch ein elektrisch isolierendes Halteelement 32 in dem Gehäuse 12 gehalten wird.

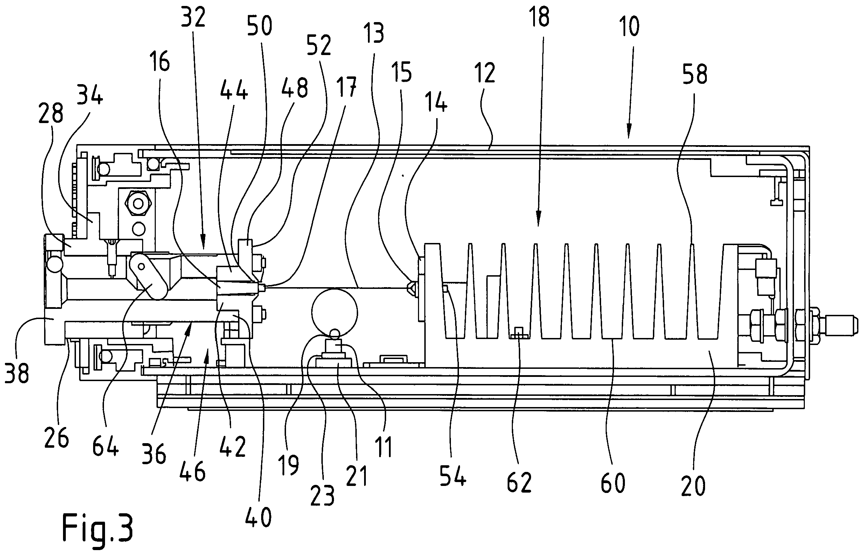

Gemäß Fig. 3 ist in der Öffnung 26 die Buchse 28 angeordnet, um das proximale Ende 22 des Lichtleiterkabels 24 in das Gehäuse 12 der Versorgungseinheit 10 einzuführen. Die Buchse 28 ist als kurzes, zylindrisches Hohlrohr mit einer ringförmigen Verbreiterung 34 ausgebildet, die dazu dient, die Buchse 28 am Gehäuse 12 zu befestigen. In der Buchse 28 ist formschlüssig eine Fassung 36 angeordnet, die ebenfalls als zylindrisches Hohlrohr ausgebildet ist. Ein erster Endbereich 38 der Fassung 36, der außerhalb der Buchse 28, d.h. außerhalb des Gehäuses 12, angeordnet ist und durch den das proximale Ende 22 des Lichtleiterkabels 24 in das Gehäuse 12 eingeführt wird, ist ringförmig verbreitert. Ein zweiter Endbereich 40 der Fassung 36 weist eine vollumfängliche Vertiefung 42 auf, deren Durchmesser größer als ein Innendurchmesser der Fassung 36 ausgebildet ist. In die Vertiefung 42 ist ein erster Abschnitt 44 einer Scheibe 46 formschlüssig aufgenommen. Ein zweiter ringförmiger Abschnitt 48 der Scheibe 46 weist einen größeren Außendurchmesser als der erste Abschnitt 44 der Scheibe 46 und als die Fassung 36 auf, so dass er über die Fassung 36 hinausragt. Die Scheibe 46 weist ferner einen zylindrischen Durchgang 50 auf, dessen Innendurchmesser etwa einem Innendurchmesser der Fassung 36 entspricht. In dem Durchgang 50 der Scheibe 46 ist das optische Lichtleitelement 16 angeordnet.

Das in Fig. 2 gezeigte Halteelement 32 ist hier als die Fassung 36 und die Scheibe 46 ausgebildet, wobei beide Bauteile aus einem elektrisch isolierenden Material, beispielswiese Kunststoff, hergestellt sind.

Die Lichtquelle 14 ist von einer Stirnseite 52 des zweiten Abschnitts der Scheibe 46 beabstandet angeordnet und rückwärtig durch mindestens eine Schraube 54 mit dem Kühlkörper 20 verbunden, wobei die mindestens eine Schraube 54 beispielsweise als M3-Gewinde ausgebildet sein kann.

Mit der Lichtquelle 14 ist über einen Lichtleitstecker 15 eine Lichtleitfaser 13 lichtleitend verbunden. Das distale Ende der Lichtleitfaser 13 ist über ein Lichtleitkupplungselement 17 mit dem optischen Lichtleitelement 16, das im zylindrischen Durchgang 50 angeordnet ist, lichtleitend verbunden.

Die Lichtleitfaser 13 wird vom Kopplungselement 19, das beispielsweise als ein metallischer Ring ausgebildet sein kann, umschlossen. Beim Ausführungsbeispiel der Fig. 2 besteht dabei keine stoffliche oder formschlüssige Verbindung zwischen dem Kopplungselement 19 und der Lichtleitfaser 13.

Das Kopplungselement 19 ist stofflich mit dem Aktor 11 verbunden, der im gezeigten Ausführungsbeispiel der Fig. 2 als Vibrationsmotor ausgeführt ist, wie er zur taktilen Signalgebung in Mobiltelephonen verwendet wird. Der Aktor 11 ist über nicht dargestellte Verbindungsleitungen mit einer Steuerung verbunden, die über Schalter und Regler an der Frontseite (nicht dargestellt) der Versorgungseinheit 10 bedient werden kann. Insbesondere kann der Aktor 11 ein- und ausgeschaltet werden, wobei auch komplexere Schaltungen zur Ansteuerung des Aktors 11 vorgesehen sein können.

Der Aktor 11 ist auf einem elastischen Trageelement 23 angeordnet, das auf einem Halteelement 21 montiert ist, welches auf der Innenseite des Gehäuses 12 des Beleuchtungssystems 10 befestigt ist. Das elastische Trageelement ist so ausgebildet, dass möglichst wenige Vibrationen des Aktors 11 auf das Gehäuse 12 übertragen werden.

Die Kühlvorrichtung 18 ist in dieser bevorzugten Ausbildung als passive Kühlung ausgebildet und arbeitet über eine Wärmekonduktion zwischen der Lichtquelle 14 und dem Kühlkörper 20. Sie weist zur Ableitung der von der Lichtquelle 14 erzeugten Wärme den wärmeleitenden Kühlkörper 20 auf, der auf der vom optischen Lichtleitelement 13 abgewandten Seite der Lichtquelle 14 angeordnet ist. Der Kühlkörper 20 weist ferner zur Erhöhung der Wärmeableitung abstehende Rippen 58 auf, die sich zu ihren freien Ende hin verjüngen. Die Rippen sind derart voneinander beabstandet, dass in Zwischenflächen 60 Schrauben 62 angeordnet sind, mit denen der Kühlkörper 20 an dem Gehäuse 12 befestigt ist.

Die von der Lichtquelle 14 erzeugte Wärme wird über den direkten Kontakt zwischen der Lichtquelle 14 und dem Kühlkörper 20 sowie über die mindestens eine Schraube 54 an den Kühlkörper 20 abgegeben. Die dem Kühlkörper 20 zugeführte Wärme verteilt sich entlang des Kühlkörpers 20 und wird von diesem an das Gehäuse 12 abgegeben.

Die Kühlvorrichtung 18 kann ebenfalls als Heatpipe ausgebildet sein, wobei der Kühlkörper 20 hierzu beispielsweise als abgeschlossener Hohlkörper aus einem wärmeleitfähigen Material, beispielsweise Aluminium, ausgebildet ist. An einer Innenseite des Hohlkörpers ist ein kapillar wirksames, dochtartiges Material angeordnet. Der Hohlkörper ist ferner mit einer Flüssigkeit unter Eigendruck oder gegebenenfalls unter einem reduzierten Druck befüllt. Wird einer Oberfläche der Heatpipe Wärme von der Lichtquelle 14 zugeführt, so beginnt die in einem Inneren der Heatpipe befindliche Flüssigkeit zu sieden und geht unter Aufnahme von Wärmeenergie zu Dampf über. Dieser Dampf verteilt sich in dem Hohlkörper und kondensiert unter Wärmeabgabe an einer kälteren Stelle einer Innenwand der Heatpipe. Das kapillar wirksame, dochtartige Material nimmt wiederum die kondensierte Flüssigkeit auf und transportiert diese zurück an eine Stelle der Heatpipe, an der Wärme zugeführt wird. Die Heatpipe bildet daher einen geschlossenen Kühlkreislauf, mit dem das Beleuchtungssystem 10 effizient gekühlt werden kann.

Das proximale Ende 22 des Lichtleiterkabels 24 kann durch einen Fixiermechanismus in der Fassung 36 gehalten werden. Hierzu ist ein Verschlusshebel 64 an der Fassung 36 angeordnet, mittels dem beispielsweise das proximale Ende 22 des Lichtleiterkabels 24 eingeklemmt werden kann.

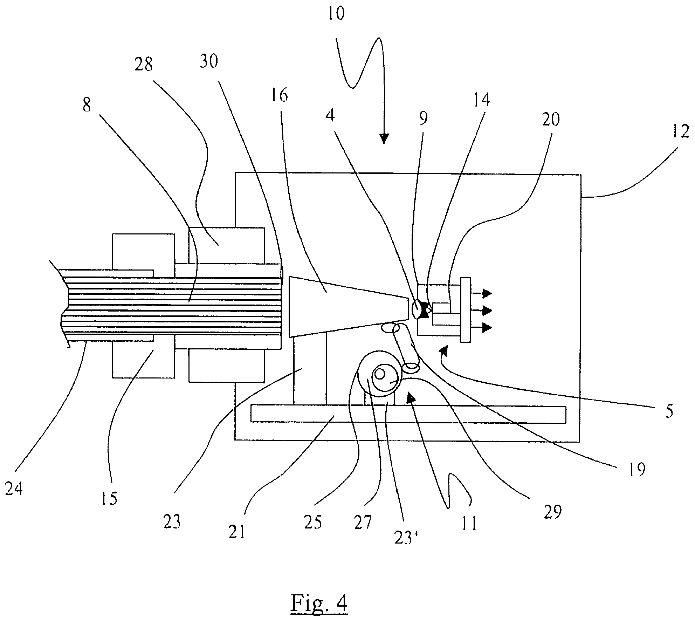

In Fig. 4 ist eine proximale Versorgungseinheit 10 dargestellt, in der bereits ein Fluoreszenzumsetzer 4 angeordnet ist. Der Fluoreszenzumsetzer 4 schließt das dreibeinige Gehäuse 9 der Anregungsstrahlenquelle 5 ab. Die Lichtquelle 14 ist als Laserchip ausgebildet, dessen aktive Fläche dem Fluoreszenzumsetzer 4 zugewandt ist und diesen beleuchtet. Der Laserchip 14 ist auf einem Kühlkörper bzw. Wärmeleitkörper 20 angeordnet. Der Wärmeleitkörper 20 ist auf dem Gehäuse 9 montiert. Die Wärme wird vom Laserchip 12 auf den Wärmeleitkörper 20 und dann an das Gehäuse 9 abgegeben und von dort über nicht dargestellte Kühlbleche und Befestigungselemente an die Versorgungseinheit 12 weitergeleitet.

Der Laserchip 14, der in diesem Beispiel Anregungslicht im blauen Spektralbereich liefert, regt den Fluoreszenzumsetzer 4 an. Die Fluoreszenzstrahlung des Fluoreszenzumsetzers 4 und das transmittierte Anregungslicht des Laserchips 14 bilden ein weißes Licht. Dieses weiße Beobachtungslicht wird in einem großen Raumwinkel (nicht dargestellt) in Richtung des Lichtleitelements 16, das in diesem Beispiel als konischer Glaskegelstumpf ausgeführt ist, abgestrahlt. Zwischen der Deckfläche des Glaskegelstumpfs 16 und dem Fluoreszenzumsetzer 4 können nicht dargestellte Ausgleichsmedien zur flexiblen Angleichung des Brechungsindex vorgesehen sein.

Der Glaskegelstumpf 16 ist auf einem sockelförmigen, elastischen Trageelement 23 aus Gummi befestigt. Das Trageelement 23 ist auf einem Halteelement 21 angeordnet, das in Form einer Metallplatte oder Platine im Gehäuse 12 der Versorgungseinheit 10 angebracht ist. Daneben befindet sich ein Aktor 11. Der Motor 27 des Aktors 11 ist auf einem weiteren elastisch Trageelement 23' gelagert. Der Drehmotor 27 weist eine Unwucht 29 auf, die an dessen Motorachse befestigt ist.

Das Kopplungselement 19, das von einer offenen, dreigliedrigen Kette aus ringförmigen Gliedern gebildet wird, ist an dem Gehäuse 25 des Motors 27 und an dem Glaskegelstumpf 16 befestigt. Dadurch wird eine Verbindung zur Kraftübertragung zwischen dem Motor 27 und dem Glaskegelstumpf 16 hergestellt. Die Drehbewegungen des Motors 27 versetzten aufgrund der Unwucht 29 das Motorgehäuse 25 in Vibrationen, die über die einzelnen Kettenglieder des Kopplungselements 19 auf den Glaskegelstumpf 16 übertragen werden, wobei die lose Ausgestaltung der Übertragungskette den Zeitverlauf der Vibrationen verändert. Die resultierenden Störungen bewirken Spannungsänderungen im Glaskegelstumpf 16, die zu entsprechenden zeitlichen Änderungen des Brechungsindex des Glaskegelstumpfs führen. Die Schwankungen des Brechungsindex modulieren die Phase des elektromagnetischen Lichts, das durch den Glaskegelstumpf 16 geleitet wird. Die zeitliche Kohärenz der Anregungsstrahlung wird mit den durch die zeitlichen Variationen des Brechungsindex induzierten Schwankungen der Phase gemittelt, wodurch dessen Kohärenz reduziert und im günstigsten Fall ausgemittelt wird.

Die Stirnseite 30 eines Glasfaserbündels 8 ist im geringen Abstand im wesentlichen parallel zur Grundfläche des Glaskegelstumpfes 16 angeordnet. Der so gebildete Zwischenraum kann wiederum mit nicht dargestellte Ausgleichsmedien zur flexiblen Angleichung des Brechungsindex aufweisen, um die Lichtverluste an dieser Kupplungsstelle der Lichtleitung möglichst gering zu halten. Das Glasfaserbündel 8 verläuft innerhalb eines Lichtleitkabels 24, dessen nicht dargestelltes distales Ende an den Lichtleiteranschluss eines Endoskops angeschlossen ist. Das Lichtleitkabel 24 weist an seinem proximalen Ende 22 einen Stecker 15, der formschlüssig in die Anschlussbuchse 28 des Gehäuses 12 der Versorgungseinheit 10 eingeführt ist.

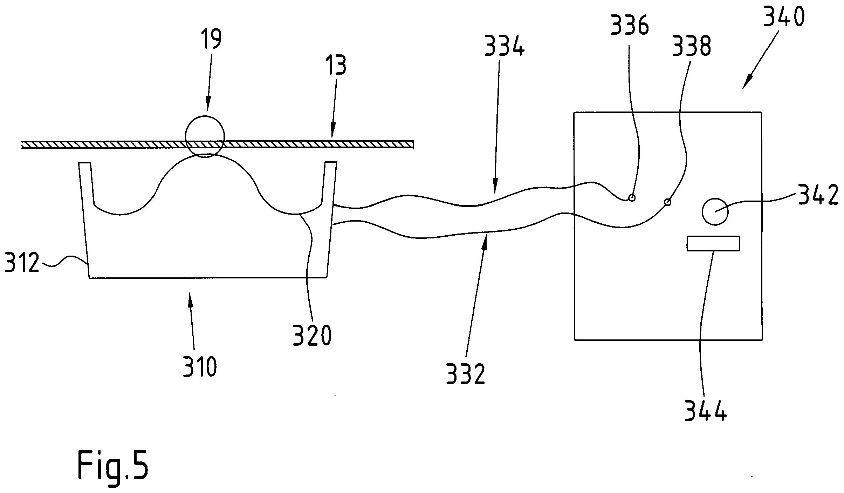

Der Aktor 11 kann, wie beispielsweise in Fig. 5 dargestellt, auch als ein handelüblicher Lautsprecher 310 ausgeführt sein. Das Lautsprechergehäuse 312 ist auf dem Gehäuse 12 des Beleuchtungssystems 10 befestigt (nicht dargestellt). Auf die Lautsprechermembran 320 ist das Kopplungselement 19 aufgeklebt, das als Kunststoffring ausgebildet ist. Durch den Kunststoffring 19 wird die Lichtfaser 13 geführt, die in lichtleitender Verbindung mit der Lichtquelle 14 steht, die als Diodenlaser ausgeführt ist (nicht dargestellt). Die Verbindungsleitungen 334 und 336 verbinden den Lautsprecher 310 mit dem Frequenzgenerator 340 über die Anschlussbuchsen 336 und 338. Der Frequenzgenerator verfügt über einen Regler 342 und eine Frequenzanzeige 344. Die Lautsprechermembran kann mit Signalen des Frequenzgenerators beaufschlagt werden, die für das menschliche Gehör nicht mehr wahrnehmbar sind. Diese Signale lenken die Lautsprechermembran 320 aus, die ihrerseits den aufgeklebten Kunststoffring 19 auslenkt, der gegen die Lichtleitfaser 13 stößt und somit Störungen des Strahlengangs in der Lichtleitfaser 13 hervorruft.

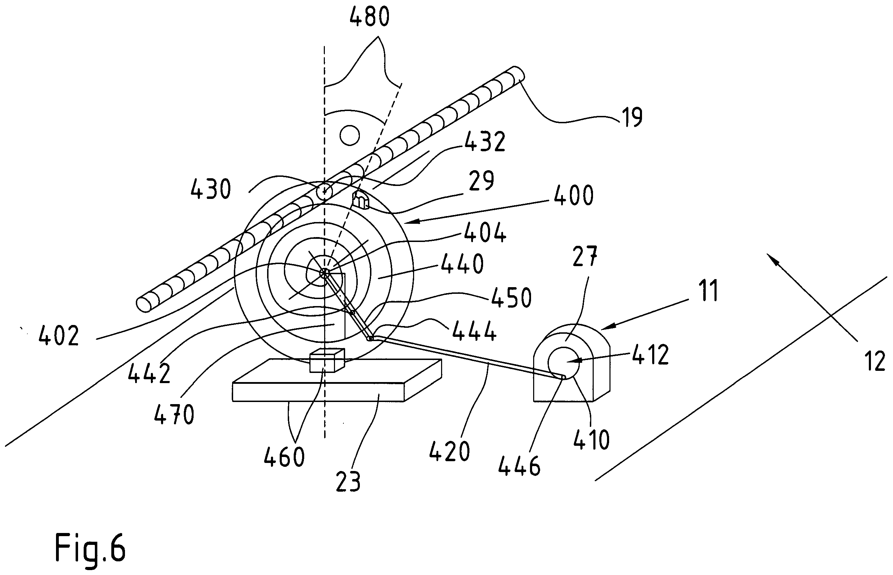

In der bevorzugten Ausgestaltung in Fig.6 dient ein metallisches Schwungrad 400 als Kopplungselement zwischen dem Aktor 11 und dem Lichtleitelement 13. Das Schwungrad ist auf der Achse 404 drehbar gelagert, die an der Halterungsstange 470 befestigt ist. Die Halterungsstange ist auf dem elastischen Trageelement 23 angeordnet.

Das Schwungrad 400 weist neben der zentralen Bohrung 402 für die Drehachse 404 eine außeraxiale Bohrung 430 auf, durch die der Lichtleiter 13 formschlüssig geführt wird. Der Lichtleiter 13 kann dabei beispielsweise durch einen geeigneten Klebstoff oder eine Vergussmasse, die zwischen das Schwungrad 400 und den Lichtleiter 13 in der Bohrung 430 eingebracht wird, zusätzlich mit dem Schwungrad stoffschlüssig verbunden sein.

Das innere Ende einer Spiralfeder 440 ist an der Drehachse 402 mit dem Schwungrad 400 verbunden. Das äußere Ende der Spiralfeder 440 greift in eine Bohrung 442 eines Hebels 450 ein, in der das äußere Ende der Spiralfeder 440 befestigt ist. Der Hebel 440 ist drehbar an der Drehachse 402 und über einen Stift 444, den eine Bohrung des Hebels formschlüssig umschließt, drehbar mit der Antriebsstange 420 verbunden.

Der Aktor 11 weist eine Exzenterscheibe 410 auf, die um die Drehachse 412 des Motors 27 rotiert. Die Antriebsstange 420 ist über eine Bohrung formschlüssig mit dem Stift 446, der auf der Exzenterscheibe 410 befestigt ist, verbunden. Durch die Übertragungskette, die aus der Antriebsstange 420, dem Hebel 450 und der Spiralfeder 440 gebildet wird, wird die Rotationsbewegung der Exzenterscheibe, die vom Motor 27 hervorgerufen wird, in eine Drehschwingung des Schwungrads 400 umgesetzt.

Auf dem elastischen Trageelement 23 ist ein Permanentmagnet 460 angeordnet. Bei Drehung des metallischen Schwungrades 400 werden durch den Permanentmagneten 460 Induktionsströme im Schwungrad hervorgerufen, deren Magnetfeld dem Magnetfeld des Permanentmagneten entgegenwirkt, wodurch die Bewegung des Schwungrads 400 gebremst wird.

Auf dem Schwungrad 400 kann ferner eine Unwucht 29 angeordnet sein.

Die Bewegung des Schwungrads 400 kann durch den Winkel θ beschrieben werden, der durch die Achsen 480 und 490 gebildet wird.

Die Bewegung des Schwungrades 400 kann durch eine Differentialgleichung vom Typ

$$\theta'' + a1 * \theta' + a2 * \theta + a3 * \sin(\theta) = a4 * \sin(\omega * t)$$

beschrieben werden, wobei der hochgestellte Strich die Ableitung nach der Zeit bedeutet. ω bezeichnet die Kreisfrequenz des Motors. Die Konstanten a1, a2, a3 und a4 sind mit dem Trägheitsmoment des Schwungrads 400, der Masse der Unwucht 29, der Erdbeschleunigung, der Federkonstante der Spiralfeder 440 und der Dämpfungskonstante, die sich aus der Bremswirkung des Permanentmagneten 460 auf das metallische Schwungrad 400 ergibt, verknüpft. Die Werte dieser Größen können so abgestimmt werden, dass sich ein chaotischer Verlauf für den Winkel θ als Funktion der Zeit ergibt, wie aus dem Buch "Nonlinear Dynamics and Chaos" von J.M.T. Thomson und H.B. Stewart , erschienen im Verlag John Wiley & Sons, 1986, entnommen werden kann.

Bei einer chaotischen Auslenkung des Schwungrads 400 wird der Verlauf des Strahlengangs innerhalb der Lichtleitfaser 13 in entsprechender Weise gestört, wodurch die Kohärenz des Lichts gestört wird und einer Entstehung von Speckles am distalen Ende des endoskopischen Systems 1 entgegengewirkt wird.

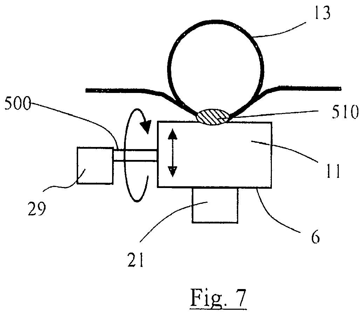

In Fig. 7 ist eine besonders einfache Ausführungsform der Kopplung eines Aktors 11 mit einem Lichtleiter 13 gezeigt. Der Aktor 11, der als Elektromotor ausgebildet ist, ist auf einem Halteelement 21, das als Sockel ausgebildet ist, angeordnet. Die Lichtleiter 13 in Gestalt einer Glasfaser ist durch einen Klebstofftropfen direkt auf dem Gehäuse 6 des Elektromotors 11 aufgeklebt. Der Elektromotor 11 versetzt durch eine Antriebswelle 500 ein Unwuchtelement 29 in Rotation, die durch den gekrümmten Pfeil in Fig. 6 angedeutet ist. Durch die Rotation des Unwuchtelements 29 wird das Gehäuse 6 des Elektromotors 11 in Schwingungen versetzt, deren Richtungen durch den Doppelpfeil angezeigt werden. Die Schwingungen werden durch die stoffschlüssige Verbindung, die durch den Klebstofftropfen hergestellt wird, unmittelbar auf die Glasfaser 13 übertragen.

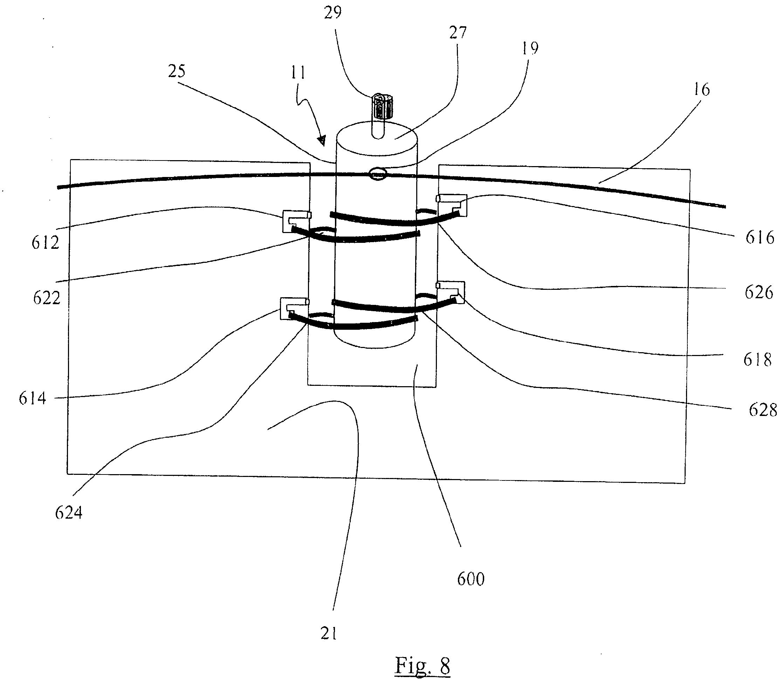

Eine besonders günstige Aufhängung des Aktors 11 ist in Fig.8 gezeigt. Das Halteelement 21 des Aktors 11 ist als Platine ausgebildet, die eine rechteckige Ausnehmung 600 aufweist. Die Platine 21 ist im nicht dargestellten Gehäuse 12 der Versorgungseinheit 10 verbaut. Die Ausnehmung 600 weist auf ihren längeren Seiten schlitzförmige Halterungen 612, 614, 616 und 618 auf, die sich paarweise gegenüberliegen. Der Motor 27 des Aktors 11 wird in der rechteckigen Ausnehmung 600 der Platine 21 durch O-Ringe 622, 624, 626 und 628 schwebend gehalten. Die O-Ringe umschlingen das Gehäuse 25 des Motors 27 und sind in den Halterungen 612, 624, 616, 618 befestigt. Die Oberfläche des Motorgehäuses 25 kann zur besseren Führung der O-Ringe Nuten aufweisen. An der Drehachse des Motors 27 ist die Unwucht 29 befestigt. Ein Kopplungselement 19 in Form eines Metallrings ist auf das Motorgehäuse 25 geschweißt. Der Metallring 19 umschließt ein Lichtleitelement 13, das als Laserfaser ausgebildet ist.

Durch die schwebende Aufhängung des Motors 27 wird die Übertragung der Motorvibrationen auf das Gehäuse 12 bzw. sonstige Komponenten der proximalen Versorgungseinheit 10 gering gehalten, wodurch unbeabsichtigte Störungen des endoskopischen Systems weitestgehend vermieden werden.

**Bezugszeichenliste:**

1 - endoskopisches System
2 - Okular
3 - Einführungsteil
4 - Fluoreszenzumsetzer
5 - Anregungsstrahlenquelle
6 - Gehäuse
7 - Kupplungsoptik
8 - Glasfaser
9 - Gehäuse der Lichtquelle
10 - Versorgungseinheit
11 - Aktor
12 - Gehäuse der Versorgungseinheit
13 - Lichtleiter
14 - Lichtquelle
15 - Lichtleitstecker
16 - optisches Lichtleitelement
17 - optisches Lichtleitkupplungselement
18 - Kühlvorrichtung
19 - Kopplungselement
20 - Kühlkörper
21 - Halteelement
22 - proximales Ende des Lichtleitkables 24
23 - elastisches Tragelement
24 - Lichtleiterkabel
25 - Motorgehäuse
26 - Öffnung
27 - Motor
29 - Unwuchtelement (Unwucht/Exzenter)
28 - Buchse
30 - Stirnfläche
32 - Halteelement
34 - ringförmige Verbreiterung
36 - Fassung
38 - erster Endbereich der Fassung 36
40 - zweiter Endbereich der Fassung 36
42 - vollumfängliche Vertiefung
44 - erster Abschnitt der Scheibe 46
46 - Scheibe
48 - zweiter Abschnitt der Scheibe 48
50 - zylindrischer Durchgang
52 - Stirnseite des zweiten Abschnitts 48 der Scheibe 46
54 - Schraube
58 - Rippen des Kühlkörpers
60 - Zwischenflächen
62 - Schrauben
64 - Verschlusshebel
310 - Lautsprecher
312 - Lautsprechergehäuse
320 - Lautsprechermembran
332 - Verbindungsleitung
334 - Verbindungsleitung
336 - Anschlussbuchse
338 - Anschlussbuchse
340 - Frequenzgenerator
342 - Regler
344 - Anzeigeelement
400 - Rad

402 - Mittelpunkt des Rades
410 - Exzenterscheibe
412 - Drehachse des Motors 27
420 - Antriebsstange
430 - Bohrung
432 - Mittelpunkt der Bohrung
440 - Spiralfeder
442 - Bohrung
444 - Stift
446 - Stift
450 - Antriebshebel
460 - Permanentmagnet
470 - Halterungsstange
480 - Orientierung des Ausschlags des Rads 400
490 - senkrechte Achse durch Radmittelpunkt und Mittelpunkt der Bohrung
500 - Antriebswelle
510 - Klebstofftropfen
600 - rechteckige Ausnehmung
612 - O-Ring
614 - O-Ring
616 - O-Ring
618 - O-Ring
622 - Halterung
624 - Halterung
626 - Halterung
628 - Halterung

**Patentansprüche**

1. Endoskopisches oder mikroskopisches System (1) mit:

    mindestens einer Lichtquelle (5,14) zur Erzeugung von zumindest teilweise kohärentem Licht, mindestens einem Lichtleitelement (13, 16), wobei die mindestens eine Lichtquelle (5, 14) und das mindestens eine Lichtleitelement (13, 16) in einer proximalen Versorgungseinheit (10) angeordnet sind,
    einer optischen Strahlungsübertragungsstrecke (8) in einem Einführungsteil (3),
    mindestens einem Aktor (11, 27, 310), der mit zumindest einem Lichtleitelement (13, 16) und/oder mit der mindestens einen Lichtquelle (5, 14) gekoppelt ist, wobei der Aktor (11, 27, 310) ausgebildet ist, um aperiodische, chaotische oder stochastische Störungen zur Reduzierung von Speckles zu erzeugen,
    **dadurch gekennzeichnet, dass** der mindestens eine Aktor (11, 27, 310) einen magnetostriktiven Wandler oder einen elektrostriktiven Wandler oder einen elektromotorischen Wandler oder einen elektrodynamischen Wandler oder eine Pockels-Zelle oder eine Kerr-Zelle oder einen Vibrationsmotor zur Erzeugung eines taktilen Alarmsignals an einem Mobilfunktelefon oder einen Linearantrieb oder einen Ventilator oder einen Lautsprecher mit vorgeschaltetem Frequenzgenerator umfasst.

2. Endoskopisches oder mikroskopisches System (1) nach Anspruch 1, **dadurch gekennzeichnet, dass** der mindestens eine Aktor (11, 27, 310) durch ein Kopplungselement (19) mit dem mindestens einen Lichtleitelement (13, 16) und/oder der mindestens einen Lichtquelle (5, 14) des endoskopischen Systems (1) stoffschlüssig und/oder formschlüssig und/oder kraftschlüssig und/oder lose gekoppelt ist.

3. Endoskopisches oder mikroskopisches System (1) nach Anspruch 2, **dadurch gekennzeichnet, dass** das Kopplungselement (19) eine lochförmige Öffnung zur Durchführung des zumindest einen Lichtleitelements (13, 16) des endoskopischen Systems (1) aufweist.

4. Endoskopisches oder mikroskopisches System (1) nach einem der Ansprüche 2 oder 3, **dadurch gekennzeichnet, dass** das Kopplungselement (19) ein Dämpfungselement (460) aufweist.

5. Endoskopisches oder mikroskopisches System (1) nach einem der Ansprüche 2 bis 4, **dadurch gekennzeichnet, dass** das Kopplungselement (19) zu Drehschwingungen anregbar ist.

6. Endoskopisches oder mikroskopisches System (1) nach einem der Ansprüche 1 bis 5, **dadurch gekennzeichnet,**
   **dass** die Lichtquelle (5,14) zur Erzeugung von zumindest teilweise kohärenten Licht, das zur Anregung von Fluoreszenzlicht geeignet ist,
   **dass** mindestens einem Fluoreszenzumsetzer (4), der zur Umwandlung in Weißlicht vorgesehen ist und geeignet ist,
   und **dass** mindestens ein Aktor (11, 27, 310) vorhanden ist, der mit dem mindestens einen Fluoreszenzumsetzer (4) insbesondere stoffschlüssig und/oder formschlüssig und/oder kraftschlüssig und/oder lose gekoppelt ist, wobei der Aktor (11, 27, 310) ausgebildet ist, um insbesondere aperiodische, chaotische, stochastische Störungen zur Reduzierung von Speckles zu erzeugen.

7. Endoskopisches oder mikroskopisches System (1) nach Anspruch 6, **dadurch gekennzeichnet, dass** der mindestens eine Fluoreszenzumwandler (4) am distalen Ende der Strahlungsübertragungstrecke (8) in dem Einführungsteil (3) angeordnet ist.

8. Endoskopisches oder mikroskopisches System (1) nach Anspruch 6, **dadurch gekennzeichnet, dass** der mindestens eine Fluoreszenzumwandler (4) in der proximalen Versorgungseinheit (10) angeordnet ist.

9. Endoskopisches oder mikroskopisches System (1) nach einem der Ansprüch 1 bis 8, **dadurch gekennzeichnet, dass** die mindestens eine Lichtquelle (5, 14) zur Erzeugung von zumindest teilweise kohärentem Licht ein Halbleiterlaser insbesondere ein Diodenlaser ist.

10. Endoskopisches oder mikroskopisches System (1) nach einem der Ansprüche 1 bis 9, **dadurch gekennzeichnet, dass** der mindestens eine Aktor (11, 27, 310) durch eine elektronische Schaltung gesteuert wird.

11. Endoskopisches oder mikroskopisches System (1) nach einem der Ansprüche 1 bis 10, **dadurch gekennzeichnet, dass** der mindestens eine Aktor (11, 27, 310) eine Unwucht (29) aufweist.

12. Endoskopisches oder mikroskopisches System (1) nach einem der Ansprüche 1 bis 11, **dadurch gekennzeichnet, dass** ein Halteelement (21) zur Lagerung des mindestens einen Aktors (11, 27, 310) in der proximalen Versorgungseinheit (10) angeordnet ist.

**Claims**

1. Endoscopic or microscopic system (1), comprising:

   at least one light source (5, 14) for producing at least partly coherent light,
   at least one light guiding element (13, 16), wherein the at least one light source (5, 14) and the at least one light guiding element (13, 16) are arranged in a proximal supply unit (10),
   an optical radiation transmission path (8) in an insertion part (3),
   at least one actuator (11, 27, 310), which is coupled to the at least one light guiding element (13, 16) and/or to the at least one light source (5, 14), wherein the actuator (11, 27, 310) is embodied to produce aperiodic, chaotic or stochastic disturbances for the purposes of reducing speckles,
   **characterized in that** the at least one actuator (11, 27, 310) comprises a magnetostrictive transducer or an electrostrictive transducer or an electromotive transducer or an electrodynamic transducer or a Pockels cell or a Kerr cell or a vibration motor for producing a tactile alarm signal at a mobile telephone or a linear drive or a ventilator or a loudspeaker with an upstream frequency generator.

2. Endoscopic or microscopic system (1) according to Claim 1, **characterized in that** the at least one actuator (11, 27, 310) is coupled in cohesive and/or interlocking and/or force-fit and/or loose fashion to the at least one light guiding element (13, 16) and/or the at least one light source (5, 14) of the endoscopic system (1) by way of a coupling element (19).

3. Endoscopic or microscopic system (1) according to Claim 2, **characterized in that** the coupling element (19) has a hole-shaped aperture for guiding through the at least one light guiding element (13, 16) of the endoscopic system (1).

4. Endoscopic or microscopic system (1) according to either of Claims 2 and 3, **characterized in that** the coupling element (19) comprises a damping element (460).

5. Endoscopic or microscopic system (1) according to any one of Claims 2 to 4, **characterized in that** the coupling element (19) can be excited to carry out rotary oscillations.

6. Endoscopic or microscopic system (1) according to any one of Claims 1 to 5, **characterized in that** the light source (5, 14) for producing at least partly coherent light, which is suitable for exciting the fluorescence light,
   **in that** at least one fluorescence converter (4), which is provided and suitable for conversion into white light,
   and **in that** at least one actuator (11, 27, 310) is present, which is coupled to the at least one fluorescence converter (4), in particular in cohesive and/or interlocking and/or force-fit and/or loose fashion, wherein the actuator (11, 27, 310) is embodied to produce disturbances, in particular aperiodic, chaotic or stochastic disturbances, for the purposes of reducing speckles.

7. Endoscopic or microscopic system (1) according to Claim 6, **characterized in that** the at least one fluorescence converter (4) is arranged at the distal ends of the radiation transmission path (8) in the insertion part (3).

8. Endoscopic or microscopic system (1) according to Claim 6, **characterized in that** the at least one fluorescence converter (4) is arranged in the proximal supply unit (10).

9. Endoscopic or microscopic system (1) according to any one of Claims 1 to 8, **characterized in that** the at least one light source (5, 14) for producing at least partly coherent light is a semiconductor laser, in particular a diode laser.

10. Endoscopic or microscopic system (1) according to any one of Claims 1 to 9, **characterized in that** the at least one actuator (11, 27, 310) is controlled by an electronic circuit.

11. Endoscopic or microscopic system (1) according to any one of Claims 1 to 10, **characterized in that** the at least one actuator (11, 27, 310) comprises an imbalance (29).

12. Endoscopic or microscopic system (1) according to any one of Claims 1 to 11, **characterized in that** a holding element (21) is arranged for mounting the at least one actuator (11, 27, 310) in the proximal supply unit (10).

**Revendications**

1. Système endoscopique ou microscopique (1) comportant :

   au moins une source lumineuse (5, 14) destinée à générer une lumière au moins partiellement cohérente, au moins un élément de guidage de lumière (13, 16), dans lequel l'au moins une source lumineuse (5, 14) et l'au moins un élément de guidage de lumière (13, 16) sont disposés dans une unité d'alimentation proximale (10),
   un chemin de transmission de rayonnement optique (8) dans une partie d'introduction (3),
   au moins un actionneur (11, 27, 310) qui est couplé à au moins un élément de guidage de lumière (13, 16) et/ou à l'au moins une source lumineuse (5, 14), dans lequel l'actionneur (11, 27, 310) est conçu pour générer des perturbations apériodiques, chaotiques ou stochastiques afin de réduire les tavelures,
   **caractérisé en ce que** l'au moins un actionneur (11, 27, 310) comprend un convertisseur magnétostrictif ou un convertisseur électro-strictif ou un convertisseur électro-motorisé ou un convertisseur électrodynamique ou une cellule de Pockels ou une cellule de Kerr ou un moteur de vibration destiné à générer un signal d'alarme tactile sur un téléphone mobile ou un entraînement linéaire ou un ventilateur, ou un haut-parleur comportant un générateur de fréquence connecté en amont.

2. Système endoscopique ou microscopique (1) selon la revendication 1, **caractérisé en ce que** l'au moins un actionneur (11, 27, 310) est couplé par l'intermédiaire d'un élément de couplage (19) à l'au moins un élément de guidage de lumière (13, 16) et/ou à l'au moins une source lumineuse (5, 14) du système endoscopique (1) par complémentarité de matériau et/ou par complémentarité de forme et/ou par complémentarité de force et/ou de manière lâche.

3. Système endoscopique ou microscopique (1) selon la revendication 2, **caractérisé en ce que** l'élément de couplage (19) comporte une ouverture en forme d'orifice destinée au passage de l'au moins un élément de guidage de lumière (13, 16) du système endoscopique (1).

4. Système endoscopique ou microscopique (1) selon l'une des revendications 2 ou 3, **caractérisé en ce que** l'élément de couplage (19) comporte un élément d'amortissement (460).

5. Système endoscopique ou microscopique (1) selon l'une des revendications 2 à 4, **caractérisé en ce que** l'élément de couplage (19) peut être excité en oscillation de rotation.

6. Système endoscopique ou microscopique (1) selon l'une des revendications 1 à 5, **caractérisé en ce que** la source lumineuse (5, 14) destinée à générer une lumière au moins partiellement cohérente, qui est appropriée pour exciter une lumière de fluorescence,
   **en ce qu'**au moins un convertisseur de fluorescence (4). qui est prévu et approprié pour effectuer une conversion en lumière blanche,
   et **en ce qu'**il est prévu au moins un actionneur (11, 27, 310) qui est couplé à l'au moins un convertisseur de fluorescence (4) notamment par complémentarité de matériau et/ou complémentarité de forme et/ou complémentarité de force et/ou de manière lâche, dans lequel l'actionneur (11, 27, 310) est réalisé de manière à générer des perturbations notamment apériodiques, chaotiques, stochastiques destinées à réduire les tavelures.

7. Système endoscopique ou microscopique (1) selon la revendication 6, **caractérisé en ce que** l'au moins un convertisseur de fluorescence (4) est disposé à l'extrémité distale du chemin de transmission de rayonnement (8) dans la partie d'introduction (3).

8. Système endoscopique ou microscopique (1) selon la revendication 6, **caractérisé en ce que** l'au moins un convertisseur de fluorescence (4) est disposé dans l'unité d'alimentation proximale (10).

9. Système endoscopique ou microscopique (1) selon l'une des revendications 1 à 8, **caractérisé en ce que** l'au moins une source lumineuse (5, 14) destinée à générer une lumière au moins partiellement cohérente est un laser à semi-conducteur, en particulier une diode laser.

10. Système endoscopique ou microscopique (1) selon l'une des revendications 1 à 9, **caractérisé en ce**

**que** l'au moins un actionneur (11, 27, 310) est commandé par un circuit électronique.

11. Système endoscopique ou microscopique (1) selon l'une des revendications 1 à 10, **caractérisé en ce que** l'au moins un actionneur (11, 27, 310) comporte un balourd (29).

12. Système endoscopique ou microscopique (1) selon l'une des revendications 1 à 11, **caractérisé en ce qu'**un élément de support (21) destiné au montage de l'au moins un actionneur (11, 27, 310) est disposé dans l'unité d'alimentation proximale (10).

Fig. 1

Fig.2

EP 2 241 921 B1

Fig.3

Fig. 4

Fig.5

Fig.6

EP 2 241 921 B1

Fig. 7

Fig. 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2005205195 A
- US 4011403 A
- EP 2187259 A1
- WO 2009034694 A

**In der Beschreibung aufgeführte Nicht-Patentliteratur**

- Speckle Reduction in Coherent Information Processing. **TOSHIAKI IWAI ; TOSHIMITSU ASAKURA.** Proceedings of the IEEE. IEEE, Mai 1996, vol. 84
- **J.M.T. THOMSON ; H.B. STEWART.** Nonlinear Dynamics and Chaos. Verlag John Wiley & Sons, 1986